# EUROPEAN PATENT APPLICATION

(11) **EP 4 818 310 A1**
(43) Date of publication of application: **07.10.2026**
(21) Application number: 24897857.9
(22) Date of filing: 07.10.2024
(51) Int. Cl.: C12N 5/077, A23L 13/00

(54) **MEDIUM ADDITIVE FOR CULTURING CULTURED MEAT, CELL CULTURE MEDIUM FOR CULTURING CULTURED MEAT COMPRISING SAME, AND METHOD FOR CULTURING CULTURED MEAT USING SAME**

(30) Priority: 01.12.2023 KR 20230172367
(71) Applicant: Hanwha Solutions Corporation, Jung-gu Seoul 04541 (KR)
(72) Inventor: KIM, Hyeong Taek, Seoul 04541 (KR); LEE, Eunji, Seoul 04541 (KR); SHIM, Minjeong, Seoul 04541 (KR); PARK, Jin-Hee, Seoul 04541 (KR)
(74) Representative: Berggren Oy
(86) International application number: PCT/KR2024/015158
(87) International publication number: WO 2025/116258

(57) **Abstract**

The present invention relates to an invention utilizing a microalgae extract as a medium additive for culturing cultured meat. More specifically, the present invention uses an extract of chlorella, which is a kind of microalgae, as a medium additive component, thereby making it possible to effectively induce cross-differentiation from muscle stem cells into adipocytes and provide cultured meat having improved flavor by inducing cells in which muscle fat is present at the same time. In addition, the use of the medium additive of the present invention replaces fetal bovine serum, which is a culture medium component for cultured meat, or is used in combination with fetal bovine serum, thereby enabling a reduction in the use of expensive fetal bovine serum and enhancing the culture effect of cultured meat, thus providing cultured meat having excellent cost effectiveness.

## Description

### TECHNICAL FIELD

The present invention relates to a medium additive including an extract of Chlorella, which is a type of microalgae, a cell culture medium for cultured meat including the same, and a method for culturing cultured meat using the same.

### BACKGROUND ART

Cultured meat refers to edible meat obtained through cell proliferation using cell engineering technology by culturing cells of living animals in a laboratory without going through the process of raising livestock. It is also called *in vitro* meat or labgrown meat because it is grown in a test tube, artificial meat because it is synthesized using stem cells by humans rather than naturally occurring ones, clean meat because it is produced in a clean production facility rather than a traditional livestock facility, and bio-artificial muscles (BAMs) because the muscle fibers that make up the cultured meat are cultivated.

Since cultured meat is produced by maturing cells until muscle tissue is formed from stem cells in order to replicate the texture of actual meat, it takes a long time and the production cost of cultured meat is very high such there is still a high wall of reality to commercialize the same.

In particular, a large amount of culture medium is used in the process of the mass proliferation of cells, and the culture medium for currently producing cultured meat includes expensive fetal bovine serum (FBS) containing various growth factors, cytokines, hormones and proteins essential for cell growth. FBS accounts for a very large proportion of the total production cost of cultured meat, and is acting as a major barrier to the commercialization and popularization of cultured meat.

In addition, fetal bovine serum, which is mainly used as an additive in the basic medium for cell growth, is collected from the blood of living calves, and thus, it is difficult to produce stably, and there is an ethical problem of taking young lives. In addition, since the amount of cattle slaughtered is fixed, the production amount is fixed, and thus, the unit price is also very high, making it unsuitable for use in cultured meat.

Accordingly, there is a need for research and product development on lowcost cultured meat culture media by replacing or using FBS in the culture media used for culturing cultured meat.

### DISCLOSURE

### TECHNICAL PROBLEM

The present invention has been completed by finding that a Chlorella extract has excellent differentiation ability from muscle cells into adipocytes or adipocyte-like cells, and culturing cultured meat can be controlled by controlling the amount of the extract used and the like. That is, the present invention aims to provide a medium additive for culturing cultured meat, a cultured meat culture medium and a cultured meat culture method using the same, by using a Chlorella extract.

Accordingly, the technical problems to be solved by the present invention are not limited to the technical problems mentioned above, and other technical problems that are not mentioned will be clearly understood by a person having ordinary skill in the technical field to which the present invention pertains from the description below.

### TECHNICAL SOLUTION

The present invention for solving the above problems relates to a medium additive for culturing cultured meat, including a Chlorella extract.

In a preferred embodiment of the present invention, the Chlorella extract may be an extract obtained by performing an extraction process on Chlorella including one or more selected from *Chlorella vulgaris*, *Chlorella pyrenidosa*, *Chlorella regularis*, *Chlorella saccharophila*, *Chlorella sorokiniana* and *Chlorella capsulata*.

In a preferred embodiment of the present invention, the extract may be a hot water extract.

In a preferred embodiment of the present invention, the hot water extract may be produced by performing a process including: step 1 of preparing a Chlorella suspension; step 2 of ultrasonically treating a suspension from step 1 to produce a Chlorella cell lysate; step 3 of performing a reflux extraction process on the Chlorella cell lysate to obtain a hot water extract; step 4 of treating the hot water extract with a base, performing a centrifugation process to obtain a supernatant, and neutralizing the supernatant to obtain a Chlorella extract; and step 5 of drying the Chlorella extract to obtain a powdered Chlorella extract.

Another object of the present invention is to provide a cell culture medium for culturing cultured meat, including the culture medium additive described above.

In a preferred embodiment of the present invention, the cell culture medium may be a serum-free medium including a basic medium and the culture medium additive, or a serum medium including a basic medium, fetal bovine serum (FBS) and the culture medium additive.

In a preferred embodiment of the present invention, the basic medium of the serum-free medium or serum medium may include one or more selected from Dulbecco's Modified Eagle's Medium (DMEM), GenGro medium, DMEM/F-12 medium, Leibovitz's L-15 medium, Iscove's Modified Dulbecco's Medium (IMDM), McCoy's 5A medium, M199 medium, Minimum Essential Medium Eagle (MEM), Minimum Essential Medium Eagle, Alpha (α-MEM), Ham's F-10 medium (Ham's F-10 Nutrient Mixture), Ham's F-12 medium (Ham's F-12 Nutrient Mixture), RPMI1640 medium, Basal Media Eagle (BME), CMRL1066 medium, Fischer's medium, Waymouth MB752/1 Medium, MCDB Medium and Williams' Medium E.

In a preferred embodiment of the present invention, the basic medium may further include one or more selected from insulin, transferrin, sodium selenite, fibroblast growth factor (FGF-2), epidermal growth factor (EGF), transforming growth factor (TGFβ3), l-ascorbate-2-phosphate trisodium salt (APS), platelet derived growth factor (PDGF-BB) and HGF (Hepatocyte Growth Factor, recombinant).

In a preferred embodiment of the present invention, the medium additive may further include, in addition to a Chlorella extract, one or more selected from phosphodiesterase inhibitor, insulin, synthetic glucocorticoid, thiazolidinedione, lecithin, antibiotics, glutamine, amino acid and vitamins.

In a preferred embodiment of the present invention, the cell culture medium for culturing cultured meat may further include fatty acids as other additives.

In a preferred embodiment of the present invention, the medium additive may include a Chlorella extract at a concentration of 0.001 to 0.500 g/L per 1L of the cell culture medium.

In a preferred embodiment of the present invention, the cell culture medium may further include one or more selected from 11.2 to 112.0 g/L of phosphodiesterase inhibitor, 0.01 to 1.00 g/L of insulin, 4.0 to 40.0 g/L of synthetic glucoticoid, 3.6 to 36.0 g/L of thiazolidinedione, 0.012 to 0.120 g/L of lecithin, antibiotics, 1.5 to 15.0 g/L of glutamine, 0.1 to 2.0 g/L of amino acid and 0.05 to 5.00 g/L of vitamins per 1 L of the cell culture medium.

In a preferred embodiment of the present invention, the cell culture medium for culturing cultured meat according to the present invention may further include fatty acids in an amount of 5.7 to 57.0 g/L per 1 L of cell culture medium.

In a preferred embodiment of the present invention, a target of culture in the cell culture medium may be muscle stem cells.

In a preferred embodiment of the present invention, the muscle stem cells may be animal muscle stem cells.

Another object of the present invention is to provide a method for culturing cultured meat using the cell culture medium described above, wherein the method performs a cell culture process to cross-differentiate animal muscle stem cells into adipocytes.

In a preferred embodiment of the present invention, the culturing cultured meat may be performed in an incubator at a temperature of 37 to 41°C and an atmosphere of 5.0 to 10.0% CO₂.

In a preferred embodiment of the present invention, the cross-differentiation efficiency increase rate of the method for culturing cultured meat according to the present invention may satisfy Equation 1 below:

[Equation 1] $2.0 % \leq \left(B-A\right) / B \times 100 % \leq 15.0 %$
wherein in Equation 1, A and B represent cross-differentiation rates of muscle stem cells into adipocytes when muscle stem cells are cultured in a cell culture medium, A is a cross-differentiation rate into adipocytes cultured using a cell culture medium not containing a Chlorella extract, and B is a cross-differentiation rate into adipocytes cultured using a cell culture medium containing a Chlorella extract.

### ADVANTAGEOUS EFFECTS

The present invention relates to a medium additive for culturing cultured meat and a cell culture medium for culturing cultured meat using the same, and by using an extract of Chlorella, which is a type of microalgae, as a medium additive component, cross-differentiation from muscle stem cells into fat cells can be effectively induced, and cells in which intramuscular fat simultaneously exists can be induced, thereby providing cultured meat with improved flavor. In addition, by using the medium additive of the present invention, it is possible to replace fetal bovine serum, which is a cultured meat culture medium component, or to use it in combination with fetal bovine serum, thereby reducing the usage of expensive fetal bovine serum and increasing the culture effect of cultured meat, thereby providing cultured meat with excellent cost efficiency.

The effects of the present invention are not limited to those mentioned above, and effects that are not mentioned will be clearly understood by those skilled in the art from the following description.

### DESCRIPTION OF DRAWINGS

FIGS. 1A and 1B show the results of cell experiments to evaluate cell viability according to Chlorella extract treatment.
FIG. 2 shows the results of measuring the efficiency of cross-differentiation of chicken muscle stem cells into adipocytes according to the composition of a cell culture medium performed in Experimental Example 2.
FIG. 3 is a digital image correlation (DIC) measurement image of cross-differentiation of muscle stem cells performed in Experimental Example 2.
FIG. 4 shows the results of measuring the efficiency of cross-differentiation of C2C12 myoblasts into adipocytes according to the composition of a cell culture medium performed in Experimental Example 3.
FIG. 5 is a digital image correlation (DIC) measurement image of cross-differentiation of C2C12 myoblasts performed in Experimental Example 3.

### MODES OF THE INVENTION

The numerical limitation ranges described in the present invention include all values within the range of start and end points, and for specific examples, the range of "20°C to 100°C" means not only the values of '20°C, 25°C, 30°C, 35°C, 40°C, 45°C, 50°C, 55°C, 60°C, 65°C, 70°C, 75°C, 80°C, 85°C, 90°C, 95°C and 100°C, but also any value between integers that are reasonable within the category of the described range, such as '20°C to 40°C, 50°C to 70°C or 80°C to 100°C.' In addition, the range of "10 minutes to 100 minutes" is meant to include all integers including, for example, "10 minutes to 20 minutes," "20 minutes to 30 minutes," "30 minutes to 40 minutes," "40 minutes to 50 minutes," "60 minutes to 70 minutes," "70 minutes to 80 minutes," "80 minutes to 90 minutes," and "90 minutes to 100 minutes," as well as any value between integers that fall within the ranges described above, such as "20 minutes to 40 minutes" and "80 minutes to 100 minutes."

In the present invention, the terms "medium", "culture medium", "culture medium", "medium composition", "culture composition" and "culture medium composition" mean a culture solution including nutrients capable of supporting the growth and survival of stem cells under *in vitro* culture conditions, and are not distinguished in this specification and may be used interchangeably.

In the present invention, "muscle stem cells" include precursor cells such as quiescent satellite cells in a growth-quiescent state and activated satellite cells called myoblasts. Muscle stem cells can differentiate into muscle cells, and in particular, muscle stem cells derived from animals such as livestock can be used for actual cultured meat production and the like, and are cells with very high utility in agricultural biology.

In the present invention, the "culturing of muscle stem cells" means cell proliferation while maintaining the differentiation ability of muscle stem cells, which is stem cell ability.

The present invention is described in more detail below.

The medium additive for culturing cultured meat of the present invention includes a Chlorella extract.

The method for producing the Chlorella extract performs a process including step 1 of preparing a Chlorella suspension; step 2 of ultrasonically treating a Chlorella suspension from step 1 to produce a Chlorella cell lysate; step 3 of performing a reflux extraction process on the Chlorella cell lysate to obtain a hot water extract; step 4 of treating the hot water extract with a base, performing a centrifugation process to obtain a supernatant, and neutralizing a supernatant to obtain a Chlorella extract; and step 5 of drying the Chlorella extract to obtain a powdered Chlorella extract.

The Chlorella suspension of step 1 may be prepared by mixing and stirring Chlorella powder and water in a weight ratio of 1:5 to 20, preferably 1:8 to 15, and more preferably 1:9 to 12.

The Chlorella constituting the chlorella raw material of step 1 may include one or more selected from *Chlorella vulgaris*, *Chlorella pyrenidosa*, *Chlorella regularis*, *Chlorella saccharophila*, *Chlorella sorokiniana* and *Chlorella capsulata*, preferably one or more selected from *Chlorella vulgaris*, *Chlorella pyrenidosa* and *Chlorella capsulata*, and more preferably one or more selected from *Chlorella vulgaris* and *Chlorella capsulata*.

Next, the ultrasonic treatment of step 2 may be performed under the conditions of a temperature of 0 to 20°C and an intensity of 10 to 50 kHz, and preferably may be performed for 30 minutes to 2 hours under the conditions of a temperature of 0 to 10°C and an intensity of 15 to 30 kHz. In this case, if the ultrasonic intensity exceeds 50 kHz, there may be a problem that the effective ingredient is decomposed and reduced due to the strong intensity, and if the ultrasonic treatment temperature exceeds 40°C, there may be a problem that the effective ingredient is denatured due to the high temperature and the content is reduced, and thus, it is advantageous to perform the ultrasonic treatment under the above conditions.

Next, the reflux extraction process of step 3 mixes Chlorella which is ultrasonically treated in step 2 with water in a weight ratio of 1:1 to 100, and preferably 1:5 to 50, to prepare a mixture solution, and then performs reflux extraction on the mixture solution using a reflux extractor at 25 to 100°C for 10 minutes to 3 hours, and preferably at 85 to 95°C for 10 minutes to 1 hour, to obtain a hot water extract.

Next, step 4 is a base treatment process, and the reason for mixing the base into the hot water extract is to separate the available active ingredients from the cell wall.

The base treatment may be performed by mixing the hot water extract and water, adding a base, and then heat treating at 80 to 105°C for 20 to 50 minutes.

In this case, for the base, one or more selected from sodium hydroxide and potassium hydroxide may be used. In this case, the concentration of the base is suitably 0.100 to 2.000 N, and preferably 0.100 to 1,000 N. If the concentration of the base is less than 0.100 N, the available effective ingredient may not be separated well into the cell wall, and if it is more than 2.000 N, there may be a problem of the effective ingredient being destroyed.

Then, after the base treatment is completed, centrifugation is performed, a supernatant is taken, and the supernatant is subjected to a neutralization process.

In addition, the neutralization process is performed by neutralizing such that the pH of the supernatant becomes pH 7.0±0.3, and preferably 7.0±0.1, and the neutralization treatment may use an acid such as hydrochloric acid.

The cell culture medium for culturing cultured meat according to the present invention uses the Chlorella extract prepared by the method described above as a medium additive, thereby reducing the amount of fetal bovine serum used in the cell culture medium or replacing the same, thereby lowering the unit cost of cultured meat production.

The cell culture medium for culturing cultured meat according to the present invention may include a serum-free medium including a basic medium and the medium additives; or a serum medium including a basic medium, fetal bovine serum (FBS) and the medium additive.

In addition, the medium additive includes a Chlorella extract, and may further include other additives in addition to the Chlorella extract.

Among the cell culture medium components, the basic medium may be arbitrarily selected from common media used in the relevant field suitable for culturing stem cells. In addition, the culture conditions may also be arbitrarily selected from appropriate conditions used in the relevant field. That is, the medium and culture conditions may be selected according to the type of cells to be cultured. The medium used for culturing is a cell culture minimum medium (CCMM), and may generally include carbon sources, nitrogen sources, and trace element components.

As a preferred example of the basic medium, the basic medium may include one or more selected from Dulbecco's Modified Eagle's Medium (DMEM), GenGro medium, DMEM/F-12 medium, Leibovitz's L-15 medium, Iscove's Modified Dulbecco's Medium (IMDM), McCoy's 5A medium, M199 medium, Minimum Essential Medium Eagle (MEM), Minimum Essential Medium Eagle, Alpha (α-MEM), Ham's F-10 medium (Ham's F-10 Nutrient Mixture), Ham's F-12 medium (Ham's F-12 Nutrient Mixture), RPMI1640 medium, Basal Media Eagle (BME), CMRL1066 medium, Fischer's medium, Waymouth MB752/1 Medium, MCDB Medium and Williams' Medium E.

In addition to the basic medium, the serum-free medium may further include one or more selected from insulin, transferrin, sodium selenite, fibroblast growth factor (FGF-2), epidermal growth factor (EGF), transforming growth factor (TGFβ3), l-ascorbate-2-phosphate trisodium salt (APS), platelet derived growth factor (PDGF-BB) and HGF (Hepatocyte Growth Factor, recombinant).

In addition, the usage amount of basic medium in the serum-free medium or serum medium is appropriately at 500 to 1,000 g/L, preferably 530 to 972 g/L, and more preferably 600 to 900 g/L per 1 L of cell culture medium. If the amount of serum-free medium is less than 500 g/L, there may be a problem of insufficient effect, and if it is more than 60 g/L, there may be a problem of cell death.

In addition, the amount of fetal bovine serum used in the serum medium is appropriately 1 to 200 g/L, and preferably 50 to 150 g/L, per 1 L of cell culture medium.

In addition, the medium additive of the serum-free medium or serum medium may include a Chlorella extract per 1 L of cell culture medium at 0.001 to 0.500 g/L, preferably 0.005 to 0.200 g/L, more preferably 0.010 to 0.080 g/L. If the concentration of the Chlorella extract is less than 0.001 g/L, the cross-differentiation enhancing effect due to the usage thereof is insignificant, and even if it is more than 0.500 mg/L, the cross-differentiation enhancing effect due to the usage thereof is insignificant or there is no further enhancing effect, and there may be a problem of cytotoxicity.

In addition, the medium additive may further include, in addition to the Chlorella extract, one or more selected from a phosphodiesterase inhibitor, insulin, synthetic glucocorticoid thiazolidinedione, lecithin, antibiotics, glutamine, amino acids and vitamins.

Among other additive components, the phosphodiesterase inhibitor may include one or more selected from 3-isobutyl-1-0methylxanthine (IBMX), sildenafil, tadalafil, vardenafil, avanafil, roflumilast, apremilast, ibudilast, cilostazol, milrinone, theophylline, aminophylline and methylxanthine.

Among other additive components, the synthetic glucocorticoid may include one or more selected from dexamethasone, prednisone, methylprednisolone, hydrocortisone and cortisol.

Among other additive components, the thiazolidinedione may include one or more selected from rosiglitazone, pioglitazone, lobeglitazone, troglitazone, ciglitazone, darglitazone, englitazone, netoglitazone and rivoglitazone.

Among other additive ingredients, the antibiotics may include one or more selected from soy lecithin, sunflower lecithin, coconut lecithin, egg yolk lecithin, quinoa lecithin and rice bran lecithin, which include lecithin.

In addition, the other additives may further include a fatty acid, and the fatty acid may further include one or more selected from canola oil, olive oil, avocado oil, peanut oil and high-oleic sunflower oil, which include oleic acid.

As a preferred embodiment of the medium additive for the cell culture medium for culturing cultured meat according to the present invention using a Chlorella extract and other additives, when the other additives include a phosphodiesterase inhibitor, insulin, a synthetic glucocorticoid, a fatty acid, a thiazolidinedione, lecithin, an antibiotic, glutamine, amino acids and vitamins, the appropriate contents of these components in the cell culture medium are as follows.

In addition, as other additives, when using a phosphodiesterase inhibitors and using IBMX as a phosphodiesterase inhibitor, IBMX is advantageous in terms of cross-differentiation when used at 11.2 to 112.0 g/L, and preferably 15.0 to 60.0 g/L, per 1 L of cell culture medium.

In addition, when using insulin as another additive, it is advantageous in terms of cross-differentiation to use the same at 0.01 to 1.00 g/L, and preferably 0.20 to 0.90 g/L, per 1 L of cell culture medium.

In addition, when using synthetic glucocorticoids as other additives and using dexamethasone as the synthetic glucocorticoid, it is advantageous in terms of cross-differentiation to use dexamethasone at 4 to 40 g/L, and preferably 5.0 to 30.0 g/L, per 1 L of cell culture medium.

In addition, when using thiazolidinedione as other additives and rosiglitazone as the thiazolidinedione, it is advantageous in terms of cross-differentiation to use rosiglitazone at 3.6 to 36 g/L, preferably 6.0 to 30.0 g/L per 1 L of cell culture medium.

In addition, when using lecithin as another additive, it is advantageous in terms of cross-differentiation to use the same at 0.012 to 0.120 g/L, and preferably 0.030 to 0.100 g/L, per 1 L of cell culture medium.

In addition, as other additives, when using antibiotics, it is advantageous in terms of stably culturing cells to use the same at 0.1 to 5.0 volume%, and preferably 0.3 to 1.2 volume%, per 1 L of cell culture medium. In addition, penicillin, streptomycin and/or gentamicin may be used as the antibiotics.

In addition, when using glutamine as other additives, it is advantageous in terms of cross-differentiation to use the same at 1.5 to 15.0 g/Lm, and preferably 2.5 to 12.0 g/L, per 1 L of cell culture medium.

In addition, when using amino acids as other additives, it is advantageous in terms of cross-differentiation to use the same at 0.1 to 2.0 g/L, and preferably 0.3 to 1.6 g/L, per 1 L of cell culture medium.

In addition, when using vitamins as other additives, it is advantageous in terms of cross-differentiation to use the same at 0.05 to 5.00 g/L, and preferably 0.20 to 3.50 g/L, per 1 L of cell culture medium.

In addition, as other additives, when using more fatty acids and using canola oil including oleic acid as a fatty acid, it is advantageous in terms of cross-differentiation to use the same at 5.7 to 57 g/L, and preferably 6.5 to 45.0 g/L, per 1 L of cell culture medium.

The target cells to be cultured in the cell culture medium for culturing cultured meat according to the present invention include muscle stem cells, mesenchymal stem cells, adipose stem cells, fibroblasts, embryonic stem cells, induced pluripotent stem cells, vascular stem cells and/or animal cells, and preferably, they may be muscle stem cells, mesenchymal stem cells, adipose stem cells, fibroblasts, embryonic stem cells, induced pluripotent stem cells or vascular stem cells derived from pigs, cattle or chickens, and more preferably, they may be muscle stem cells derived from chickens.

As a preferred embodiment of the method for culturing meat using the cell culture medium for culturing cultured meat according to the present invention, a cell culture process for cross-differentiating muscle stem cells into adipocytes may be performed.

To explain more specifically, the cell culture process uses the cell culture medium for cultured meat culture described above for culture. In addition, the cultured meat culture conditions are advantageous in terms of cross-differentiation when the culture is performed in an incubator at 37 to 41°C and 5.0 to 10.0% CO₂ atmosphere, and preferably in an incubator at 38 to 40°C and 5.0 to 7.0% CO₂ atmosphere.

The method for culturing cultured meat using a cell culture medium including the medium additive of the present invention has an excellent cross-differentiation efficiency of muscle cells or muscle stem cells into adipocytes. The cross-differentiation efficiency increase rate of the method for culturing cultured meat according to the present invention may satisfy Equation 1. 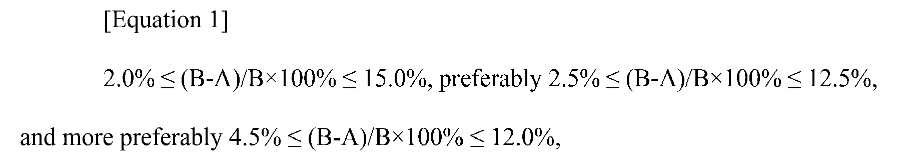 [Equation 1] 2.0% ≤ (B-A)/B×100% ≤ 15.0%, preferably 2.5% ≤ (B-A)/B×100% ≤ 12.5%, and more preferably 4.5% ≤ (B-A)/B×100% ≤ 12.0%,

In Equation 1, A and B represent the cross-differentiation rates of muscle stem cells into adipocytes when culturing muscle stem cells in a cell culture medium. A is the cross-differentiation rate of muscle stem cells into adipocytes when cultured using a cell culture medium that does not contain Chlorella extract, and B is the cross-differentiation rate of muscle stem cells into adipocytes when cultured using a cell culture medium containing Chlorella extract.

Using the cell culture medium for culturing cultured meat described above, high-quality cultured meat may be provided at high economic feasibility.

In the present invention, the cultured meat may include muscle cells and intramuscular fat at the same time.

Furthermore, the present invention may provide a food composition including the cultured meat described above.

The present invention is described in detail through preferred examples, and the following examples are intended to illustrate the present invention, but the present application is not limited to the following examples.

### [Example]

### Example 1: Preparation of Chlorella extract (culture medium additive)

As Chlorella, *Chlorella vulgaris* (DAESANG chlorella powder) was prepared.

Next, the Chlorella powder and distilled water were mixed in a weight ratio of 1:10 to prepare a suspension, which was then placed in an ultrasonic processor, and ultrasonic treatment was performed for 1 hour under the conditions of an output of 700 kW, a temperature of 4°C and an ultrasonic intensity of 20 kHz to obtain a Chlorella cell lysate with the cell walls disrupted.

Next, the Chlorella cell lysate was subjected to reflux extraction using a reflux extractor at 95°C for 30 minutes to obtain a hot water extract.

Next, a mixture was prepared by mixing the hot water extract and the same amount of distilled water used in preparing the suspension, and then, a base was added to the mixture such that the concentration of 0.25 N sodium hydroxide became 0.125 N, and then, the mixture was placed in an autoclave and heat-treated at 100°C for 30 minutes to perform base treatment.

Next, the base-treated mixture was cooled and centrifuged at 3,900 rpm for 20 minutes, and only the supernatant was collected.

Next, the supernatant was neutralized to pH 7.0±0.1 using hydrochloric acid to obtain a Chlorella extract.

Next, the Chlorella extract was freeze-dried to obtain a powdered Chlorella extract.

### Production Example 1: Production of cell culture medium for culturing cultured meat

The Chlorella extract prepared in Example 1 was used as an additive to a culture medium to product a cell culture medium for cultured meat cross-differentiation having the following composition.

The cell culture medium for cultured meat cross-differentiation includes, as a basic medium, 900 g/L of Dulbecco's Modified Eagle's Medium High glucose (DMEM), 0.01 g/L of insulin, 100 g/L of FBS, the Chlorella extract of Example 1 as a medium additive, and as other additives of the medium additive, 11.2 g/L of 3-isobutyl-1-0methylxanthine (IBMX), 0.1 g/L of insulin, 4.0 g/L of dexamethasone, which is a synthetic glucocorticoid, and 3.6 g/L of rosiglitazone, which is a thiazolidinedione.

In this case, the culture medium additive (the Chlorella extract of Example 1) was added at 0.001 g/L, 0.01 g/L, 0.03 g/L, 0.05 g/L, 0.07 g/L and 0.1 g/L, respectively, to prepare separate culture media.

### Comparative Production Example 1

Cell culture media having the same composition as in Production Example 1 were produced, except that instead of the Chlorella extract of Example 1, chlorella powder from Company D (containing 1 wt% of total chlorophyll) was used as a medium additive at concentrations of 0.001 g/L, 0.01 g/L, 0.03 g/L, 0.05 g/L, 0.07 g/L and 0.1 g/L.

### Comparative Production Example 2

Cell culture media having the same composition as in Production Example 1 were prepared, except that instead of the Chlorella extract of Example 1, commercially available chlorella powder (Shaanxi Ruiwo Phytochem Growth Factor Chlorella extract CGF OD1200) was used as a medium additive at concentrations of 0.001 g/L, 0.01 g/L, 0.03 g/L, 0.05 g/L, 0.07 g/L and 0.1 g/L.

### Comparative Production Example 3 (Positive Control Group)

A cell culture medium having the same composition as in Production Example 1 was prepared, except that a cell culture medium without using a medium additive (Chlorella extract of Example 1) was prepared as a control.

**[Table 1]**

| Classification (g/L) | | Negative control group | Comparative Production Example 3 (Positive control group) | Production Example 1, Comparative Production Examples 1 to 2 |
|---|---|---|---|---|
| Base medium | DMEM | 900 | 875.48 | 875.38 |
| | Insulin | 0 | 0.01 | 0.01 |
| Fetal bovine serum (FBS) | | 100 | 100 | 100 |
| Medium additive | Chlorella extract | 0 | 0 | 0.001 ~ 0.100 |
| | IBMX | 0 | 11.2 | 11.2 |
| | Dexamethasone | 0 | 4.0 | 4.0 |
| | Rosiglitazone | 0 | 3.6 | 3.6 |
| | Lecithin | 0 | 0.012 | 0.012 |

### Experimental Example 1: Cytotoxicity test of cell culture media

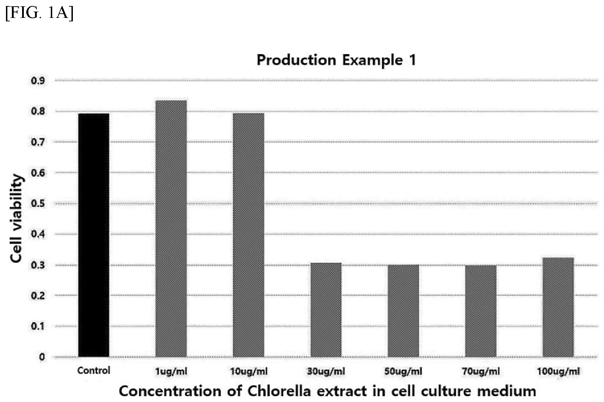

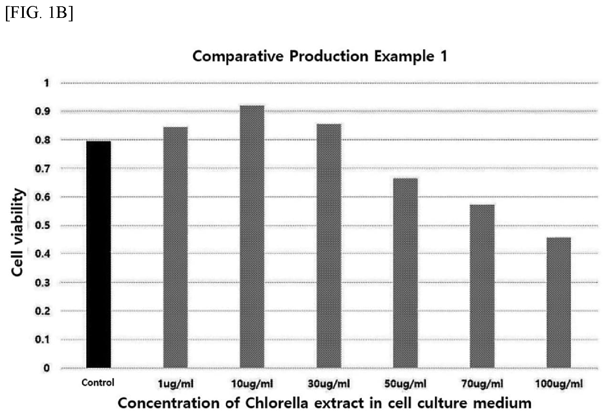

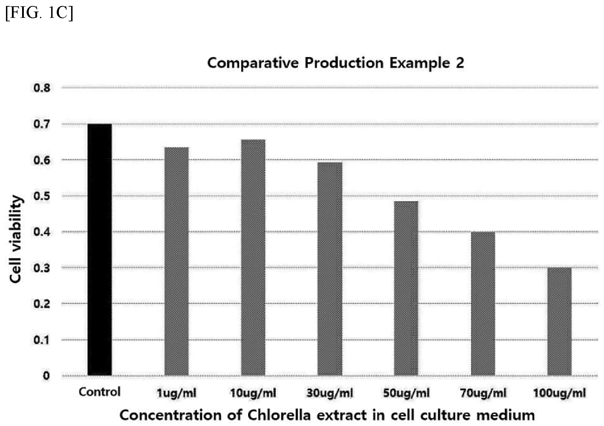

The cytotoxicity (or cell viability) of chicken muscle stem cells to cell culture media at different concentrations of the Chlorella extracts produced in Production Example 1 and Comparative Production Examples 1 and 2 was measured by performing a CCK8 assay, and the results are shown in FIG. 1A (Production Example 1), FIG. 1B (Comparative Production Example) and FIG. 1C (Comparative Production Example 2).

Looking at FIGS. 1A to 1C, it was confirmed that not only Production Example 1 but also Comparative Production Examples 1 to 2 exhibited cytotoxicity when the concentration of the Chlorella extract in the cell culture medium was 30 mg/L (0.03 g/L) or higher. In addition, it was confirmed that the cell viability decreased in a concentration-dependent manner at a concentration of 100 mg/L (0.1 g/L) or higher. Therefore, the cross-differentiation rate measurement experiment was conducted at a concentration of 10 mg/L (0.01 g/L), which is a level that does not cause toxicity to the cells.

### Experimental Example 2: Experiment to measure cross-differentiation rate of chicken muscle stem cells into adipocytes

An experiment was conducted to measure the cross-differentiation rate into adipocytes by culturing chicken muscle stem cells using cell culture media for culturing cultured meat including 10 mg/L (0.01 g/L) of the Chlorella extracts prepared in Production Example 1 and Comparative Production Examples 1 to 2 and the cell culture media of Comparative Production Examples 1 to 2, respectively.

The chicken muscle stem cells were obtained from cells extracted from the chest and legs of chickens hatched on the 17th, 18th, and 19th days using fertilized eggs cultured in a sterile laboratory.

The experimental method was to remove the medium from the adipocytes cultured by cross-differentiation from chicken muscle stem cells, add DPBS to wash the cells, mix thoroughly, and then remove the medium.

Next, 4% formalin solution was added at room temperature and left for at least 1 hour. Next, after removing formalin, it was washed 3 times with distilled water, isopropanol at a concentration of 60 volume% was added, and then it was left for 5 minutes, isopropanol was removed, and it was dried completely. Oil Red O Working Solution was added thereto, and it was left for 10 minutes at room temperature. Oil Red O solution was removed and immediately, 3-distilled water was added thereto. After observing under a microscope, it was confirmed that the cells were stained, and images were photographed. The water was discarded, and the flask was dried. Isopropanol at a concentration of 100% by volume was added thereto, and it was shaken slowly for 10 minutes to completely dissolve Oil Red O. Absorbance was measured at 490 nm using a microplate reader, and absorbance was measured again at 630 nm to correct the absorbance at 490 nm.

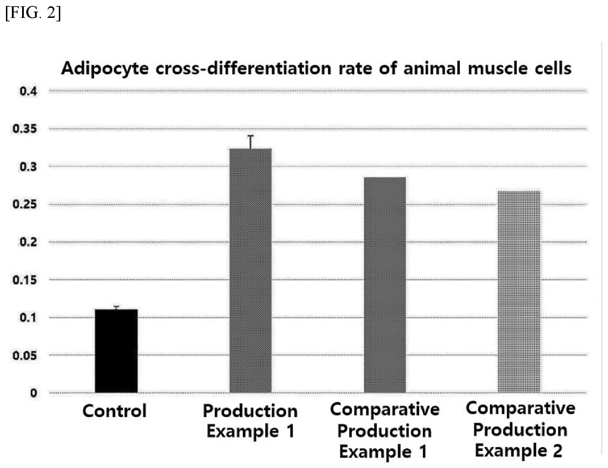

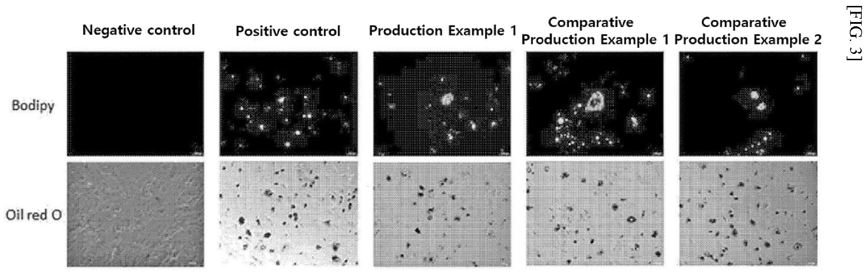

In addition, the results are shown in Table 3 and FIG. 2 below, and FIG. 3 shows the DIC (Digital image correlation) measurement images of the crossdifferentiated muscle stem cells of the positive control group and Production Example 1.

In addition, the cross-differentiation increase rate in Table 2 represents the cross-differentiation increase rate compared to the cross-differentiation rate of the positive control group.

The negative control group and positive control group in Table 2 were tested using a medium having the composition in Table 2.

**[Table 2]**

| Classification | Negative control group | Positive control group | Production Example 1 | Comparative Production Example 1 | Comparative Production Example 2 |
|---|---|---|---|---|---|
| Cross-differentiation rate of muscle stem cells into adipocytes | - | 24% | 29% | 26% | 24% |
| Cross-differentiation increase rate | - | - | 5% | 1% | 0% |

Looking at Table 2 and FIG. 2 above, it can be confirmed that the cell culture medium of Production Example 1 had the highest cross-differentiation rate, and Comparative Production Example 2 did not have a cross-differentiation enhancement effect despite including a Chlorella extract. In addition, it was confirmed that Production Example 1 had a cross-differentiation enhancement effect that was 5 times higher than that of the cell culture medium of Comparative Production Example 1.

### Experimental Example 3: Experiment to measure cross-differentiation rate of C2C12 myoblasts

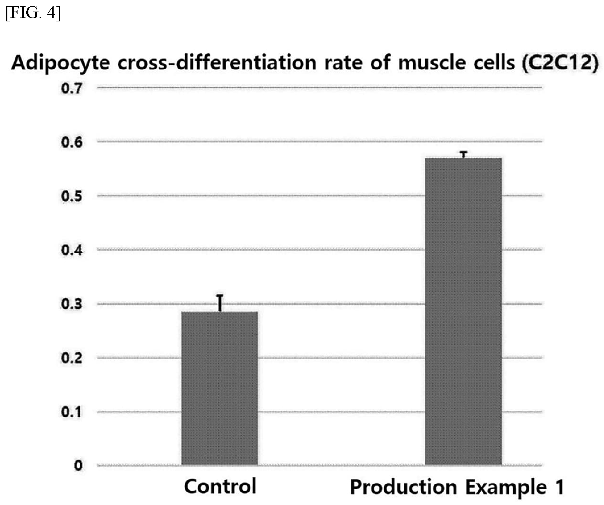

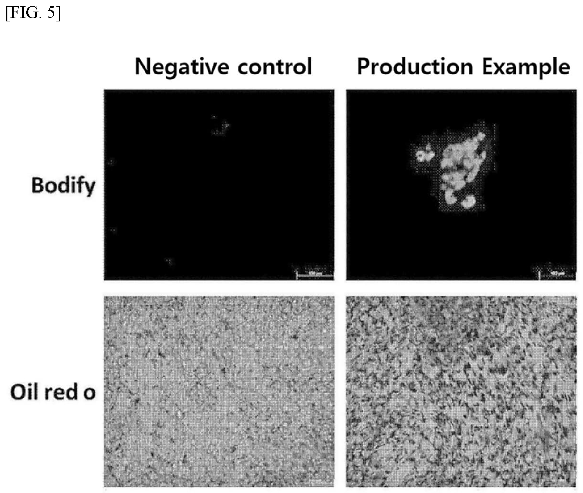

An experiment was performed to measure the cross-differentiation rate into adipocytes by culturing C2C12 myoblasts in a cell culture medium for culturing cultured meat including 10 mg/L (0.01 g/L) of the Chlorella extract produced in Production Example 1. The experimental method and conditions were the same as in Experimental Example 2, and the results are shown in Table 3 and FIG. 4 below, and FIG. 5 shows DIC measurement images of the cross-differentiation of C2C12 myoblasts of the positive control group and Production Example 1.

In addition, the positive control group in Table 3 below is an experiment conducted using a medium having the composition in Table 2 above.

**[Table 3]**

| Classification | Comparative Production Example 3 (positive control group) | Production Example 1 |
|---|---|---|
| Cross-differentiation rate into adipocytes | 28% | 57% |
| Cross-differentiation increase rate | - | 29% |

Through the above examples and experimental examples, it was confirmed that the Chlorella extract produced by the method suggested in the present invention had excellent cell proliferation and culture effects as an additive to a medium for culturing cultured meat, and by using the same as a medium for culturing cultured meat, the cross-differentiation rate of muscle cells or muscle stem cells into adipocytes was very excellent.

## Claims

1. A medium additive for culturing cultured meat, comprising a Chlorella extract.

2. The medium additive of claim 1, wherein the Chlorella extract is an extract obtained by performing an extraction process on Chlorella comprising one or more selected from *Chlorella vulgaris*, *Chlorella pyrenidosa*, *Chlorella regularis*, *Chlorella saccharophila*, *Chlorella sorokiniana* and *Chlorella capsulata*.

3. The medium additive of claim 2, wherein the extract is a hot water extract.

4. The medium additive of claim 2, wherein the hot water extract is produced by performing a process comprising:
step 1 of preparing a Chlorella suspension;
step 2 of ultrasonically treating a suspension from step 1 to produce a Chlorella cell lysate;
step 3 of performing a reflux extraction process on the Chlorella cell lysate to obtain a hot water extract;
step 4 of treating the hot water extract with a base, performing a centrifugation process to obtain a supernatant, and neutralizing the supernatant to obtain a Chlorella extract; and
step 5 of drying the Chlorella extract to obtain a powdered Chlorella extract.

5. A cell culture medium for culturing cultured meat, comprising the culture medium additive of claim 1.

6. The cell culture medium of claim 5, wherein the cell culture medium is a serum-free medium comprising a basic medium and the culture medium additive, or a serum medium comprising a basic medium, fetal bovine serum (FBS) and the culture medium additive.

7. The cell culture medium of claim 6, wherein the basic medium of the serum-free medium or serum medium comprises one or more selected from Dulbecco's Modified Eagle's Medium (DMEM), GenGro medium, DMEM/F-12 medium, Leibovitz's L-15 medium, Iscove's Modified Dulbecco's Medium (IMDM), McCoy's 5A medium, M199 medium, Minimum Essential Medium Eagle (MEM), Minimum Essential Medium Eagle, Alpha (α-MEM), Ham's F-10 medium (Ham's F-10 Nutrient Mixture), Ham's F-12 medium (Ham's F-12 Nutrient Mixture), RPMI1640 medium, Basal Media Eagle (BME), CMRL1066 medium, Fischer's medium, Waymouth MB752/1 Medium, MCDB Medium and Williams' Medium E.

8. The cell culture medium of claim 6, wherein the basic medium further comprises one or more selected from insulin, transferrin, sodium selenite, fibroblast growth factor (FGF-2), epidermal growth factor (EGF), transforming growth factor (TGFβ3), l-ascorbate-2-phosphate trisodium salt (APS), platelet derived growth factor (PDGF-BB) and HGF (Hepatocyte Growth Factor, recombinant).

9. The cell culture medium of claim 6, wherein the medium additive further comprises, in addition to a Chlorella extract, one or more selected from phosphodiesterase inhibitor, insulin, synthetic glucocorticoid, thiazolidinedione, lecithin, antibiotics, glutamine, amino acid and vitamins.

10. The cell culture medium of claim 6, wherein the medium additive comprises a Chlorella extract at a concentration of 0.001 to 0.500 g/L per 1L of the cell culture medium.

11. The cell culture medium of claim 10, wherein the cell culture medium comprises 11.2 to 112.0 g/L of phosphodiesterase inhibitor, 0.01 to 1.00 g/L of insulin, 4.0 to 40.0 g/L of synthetic glucoticoid, 3.6 to 36.0 g/L of thiazolidinedione, 0.012 to 0.120 g/L of lecithin, antibiotics, 1.5 to 15.0 g/L of glutamine, 0.1 to 2.0 g/L of amino acid and 0.05 to 5.00 g/L of vitamins per 1 L of the cell culture medium.

12. The cell culture medium according to any one of claims 5 to 11, wherein a target of culture in the cell culture medium is muscle stem cells.

13. The cell culture medium of claim 12, wherein the muscle stem cells are animal muscle stem cells.

14. A method for culturing cultured meat using the cell culture medium according to any one of claims 5 to 11, wherein the method performs a cell culture process to cross-differentiate animal muscle stem cells into adipocytes.

15. The method of claim 14, wherein the culturing cultured meat is performed in an incubator at a temperature of 37 to 41°C and an atmosphere of 5.0 to 10.0% CO₂.

16. The method of claim 14, wherein the cross-differentiation efficiency increase rate satisfies Equation 1 below: [Equation 1] $2.0 % \leq \left(B-A\right) / B \times 100 % \leq 15.0 %$ wherein in Equation 1, A and B represent cross-differentiation rates of muscle stem cells into adipocytes when muscle stem cells are cultured in a cell culture medium, A is a cross-differentiation rate into adipocytes cultured using a cell culture medium not containing a Chlorella extract, and B is a cross-differentiation rate into adipocytes cultured using a cell culture medium containing a Chlorella extract.
